# EUROPÄISCHE PATENTSCHRIFT

(11) **EP 0 479 723 B1**
(45) Veröffentlichungstag und Bekanntmachung des Hinweises auf die Patenterteilung: **27.08.1997**
(21) Anmeldenummer: 91810750.9
(22) Anmeldetag: 24.09.1991
(51) Int. Cl.: A61L 2/00, G02C 13/00, A61L 2/08

(54) **Verfahren zur Oberflächenreinigung und/oder Sterilisation von optischen Bauteilen, insbesondere von Kontaklinsen**
Process for cleaning the surface of optical devices and for their sterilisation, specially for contact lenses
Procédé pour le nettoyage et/ou la stérilisation des surfaces d'appareils optiques, notamment des lentilles de contact

(30) Priorität: 02.10.1990 CH 3208/90
(43) Veröffentlichungstag der Anmeldung: 08.04.1992
(73) Patentinhaber: Novartis AG, 4058 Basel (CH)
(72) Erfinder: Hagmann, Peter, Dr., W-8759 Hösbach-Bahnhof (DE); Höfer, Peter, W-8750 Aschaffenburg (DE); Herbrechtsmeier, Peter, Dr., W-6240 Königstein (DE)

(56) Entgegenhaltungen:
- FR-A- 2 539 030
- US-A- 3 852 032
- US-A- 4 115 280
- US-A- 4 223 782

## Beschreibung

Die Erfindung betrifft ein Verfahren zur Oberflächenreinigung und/oder Sterilisation von optischen Bauteilen, insbesondere von Kontaktlinsen gemäss Oberbegriff des Patentanspruchs 1.

Die Herstellung von optischen Bauteilen, wie beispielsweise Linsen, Prismen oder Planglasflächen, erfolgt in einer Vielzahl von einzelnen Fertigungsschritten. Dabei tragen praktisch jeder Fertigungsschritt und die dabei erforderlichen Manipulationen und Handhabungen der optischen Bauteile zu Verunreinigungen der Oberflächen der Bauteile bei. Beispielsweise werden Linsen in den einzelnen Dreh-, Fräs-, Schleif- und Polierschritten auf entsprechenden Tragkörpern befestigt. Beispielsweise werden die Linsen dabei mit Hilfe von Wachs auf die Halter aufgekittet. Nach der Behandlung werden die Linsen wieder von den Haltern abgenommen und sorgfältig von den Wachsrückständen gereinigt. Zur Reinigung der Linsen werden bevorzugt Reinigungsflüssigkeiten eingesetzt. In vielen einzelnen Reinigungsschritten, welche unter anderem Eintauchen der Linse in die Reinigungsflüssigkeit, Abreiben, Ultraschallbehandlung, Abspülen und schliesslich Trocknen der Linse umfassen, werden die Verunreinigungen von den Linsenoberflächen entfernt.

Trotz dieses Aufwands für die Reinigung ist das Ergebnis oft nicht befriedigend, da nicht alle Rückstände vollständig von den Linsenoberflächen entfernt werden können sondern in der Form eines dünnen Films an den Oberflächen oder zumindest an Teilen davon haften bleiben. Diese Rückstände können aber dann zu einer Beeinträchtigung der Funktionseigenschaften der optischen Bauteile führen. Auch enthalten die Reinigungsflüssigkeiten vielfach umweltbelastende Stoffe. Bei ihrer Handhabung kann es trotz umfangreicher Vorkehrungen beispielsweise hinsichtlich Absaugungen und Schutz vor unmittelbarem Kontakt zu unerwünschten Allergien beim Bedienungspersonal kommen.

Bei Kontaktlinsen handelt es sich bei diesen fertigungsbedingten Rückständen oft um dünne Wachsfilme mit einer Schichtdicke von im Regelfall weniger als 1 µm. Bei den Rückständen kann es sich auch um Polier- und Reinigungsmittelreste handeln oder auch vielfach um Fettrückstände von Fingerabdrücken. Derartige Rückstände können bei Kontaktlinsen zu erheblichen Benetzungsproblemen und damit zu einer Herabsetzung der Verträglichkeit der Kontaktlinsen führen. Diese Rückstände können aber auch als Haftzentren für Ablagerungen auf den Kontaktlinsen wirken und müssen daher entfernt werden. Vielfach werden die Kontaktlinsen auch sterilisiert, um beim Gebrauch der Kontaktlinsen schädliche Nebenwirkungen durch Keime auf den Linsenoberflächen auszuschliessen, bevor sie in keimfreier, unkonservierter Lösung verpackt werden. Die Sterilisation erfolgt dabei bislang beispielsweise nach Verfahren mit Heissluft oder feuchter Hitze, vielfach werden auch mikrobizide Gase verwendet. Diese Verfahren sind aber nur dann anwendbar, wenn die Sterilisationsbedingungen nicht zu einer Schädigung der Kontaktlinse, bzw. ganz allgemein bei Sterilisationen zu Schädigungen des Materials, aus welchem die optischen Bauteile gefertigt sind, führen. Eine Behandlung mit Heissluft oder feuchter Hitze bei Temperaturen von über 160°C kommt beispielsweise für viele Kontaktlinsenmaterialien nicht in Frage, da dadurch das Linsenmaterial beschädigt werden kann oder geometrische Deformationen eintreten können. Bei der Anwendung der Gassterilisation, üblicherweise mit Aethylenoxid, kommt es oftmals zu einer unerwünschten Speicherung des Gases im Linsenmaterial. Insbesondere bei Intraokularlinsen kann es aufgrund von Rückständen des Sterilisationsgases zu Komplikationen kommen, die vielfach sogar zur Explantation der Linse führen können.

In der US-A-4,223,782 ist eine herkömmliche Vorrichtung zur Reinigung und zum anschliessenden Spülen von Kontaktlinsen mit Hilfe einer Reinigungs- und einer Spülflüssigkeit beschrieben, wie sie beispielsweise von Kontaktlinsenträgem zur täglichen Linsenbehandlung eingesetzt wird. Zur Entfernung von aus dem Herstellungsprozess an der Oberfläche einer Kontaktlinse anhaftenden Verunreinigungen ist diese Vorrichtung jedoch ungeeignet.

In der US-A-3,852,032 ist ein Verfahren zur Sterilisation von weichen Kontaktlinsen mit Hilfe von ultravioletter Strahlung beschrieben. Das beschriebene Verfahren ist allerdings nur bei Kontaktlinsen anwendbar, deren Linsenmaterialien UV-Stabilisatoren enthalten, welche einen Teil der Energie der ultravioletten Strahlung beispielsweise absorbieren, um eine Schädigung des Linsenmaterials zu vermeiden.

In der FR-A-2,539,030 ist ein weiteres Verfahren zur Sterilisation von Kontaktlinsen mit Hilfe von ultravioletter Strahlung und eine dazu geeignete Vorrichtung beschrieben. Um einer Schädigung des Linsenmaterials durch die UV-Strahlung mit einer Wellenlänge von 273,7 nm vorzubeugen, wird die Energiedichte der UV-Strahlung relativ gering gehalten, typischerweise um 0,04 J/cm². Für eine zuverlässige Entfernung von Verunreinigungen von der Oberfläche einer Kontaktlinse ist diese Behandlung allerdings nicht geeignet.

In der US-A-4,115,280 ist eine Vorrichtung zur Veränderung der biologischen und chemischen Aktivität von Makromolekülen beschrieben. Die Vorrichtung umfasst eine Quelle für infrarote Strahlung, mit deren Hilfe ein zu behandelndes Objekt bestrahlt wird. Für die Entfernung von Verunreinigungen von der Oberfläche eines optischen Bauteils, beispielsweise einer Kontaktlinse, ist die Vorrichtung nicht geeignet.

Es besteht daher die Aufgabe ein Verfahren zur Reinigung und zur Sterilisation von Oberflächen von optischen Bauteilen, insbesondere von Kontaktlinsen, zu schaffen, welches die erwähnten Nachteile eliminiert. Insbesondere soll die Oberflächenreinigung und die Sterilisation der optischen Bauteile, insbesondere der Kontaktlinsen, in einem Arbeitsgang erfolgen. Das Verfahren soll einfach und wirtschaftlich durchführbar sein und für eine einfache Automatisierung des Ablaufs geeignet sein. Ueberdies sollen umweltbelastende Reinigungsmittel und Sterilisationsgase vermieden werden. Die Oberflächen der optischen Bauteile sollen nach erfolgter Behandlung weitgehend rückstandsfrei sein und auf den Oberflächen vorhandene Mikroorganismen entweder abgetötet, inaktiviert oder entfernt sein.

Diese und noch weitere Aufgaben werden durch ein Verfahren, wie es durch die Merkmale Patentanspruch 1 charakterisiert ist, gelöst. Weitere bevorzugte Verfahrensvarianten sind Gegenstand der abhängigen Patentansprüche.

Im folgenden wird das erfindungsgemässe Verfahren anhand der Zeichnung näher erläutert. Es zeigen
- Fig. 1: eine schematische Prinzipdarstellung einer Vorrichtung zur Durchführung des erfindungsgemässen Verfahrens und
- Fig. 2: eine Kontaktlinse mit Verunreinigungen und mit strichliert angedeutetem Frontflächenverlauf nach der erfindungsgemässen Behandlung.

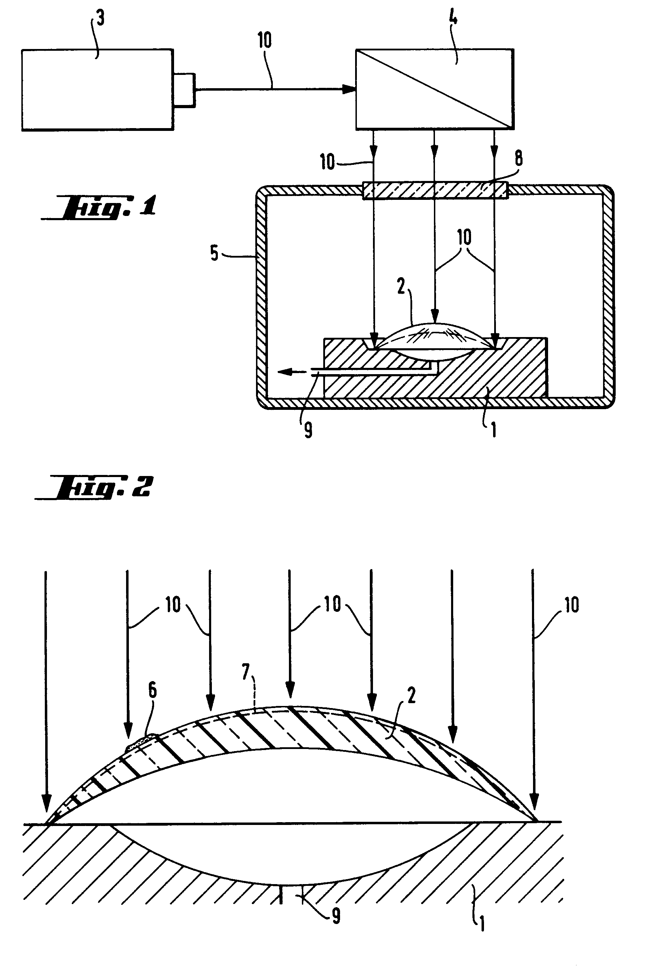

Fig. 1 zeigt eine schematische Prinzipdarstellung einer beispielsweisen Vorrichtung zur Durchführung des erfindungsgemässen Verfahrens. Insbesondere handelt es sich dabei um eine Vorrichtung zur Behandlung von Kontaktlinsen. Es versteht sich, dass die schematisch angedeutete Vorrichtung für die Behandlung von optischen Linsen, Prismenflächen oder Brillengläsern usw. gegebenenfalls modifizert sein kann. Gemäss der Darstellung umfasst die Vorrichtung im wesentlichen eine Halterung 1 für ein zu behandelndes optisches Bauteil 2, hier eine Kontaktlinse, und eine Strahlungsquelle 3 für elektromagnetische Strahlung 10 zusammen mit einer optischen Anordnung 4. Bei der Strahlungsquelle 3 für die elektromagnetische Strahlung 10 handelt es sich um einen Laser, vorzugsweise um einen sogenannten Excimer-Laser (Excimer von Excited Dimer). Die optische Anordnung 4 umfasst je nach Anwendungsfall Fokussiermittel und/oder Strahlaufweitmittel und gegebenenfalls Strahlablenkmittel. Die Halterung 1 kann beispielsweise als umlaufendes Band ausgebildet sein. An einer nicht dargestellten Beladestation werden die Linsen 2 in die Halterungen 1 eingelegt und beispielsweise durch einen geringen Unterdruck fixiert. In Fig. 1 ist dies durch die Unterdruckleitung 9 symbolisiert Die Halterung 1 wird vorzugsweise innerhalb eines Behältnisses 5 angeordnet. Das Behältnis 5 weist ein oder mehrere für die verwendete elektromagnetische Strahlung transparente Fenster 8 auf, es kann aber auch zur Gänze transparent ausgebildet sein. Ueber eine Ein- und Ausbringschleuse werden die Linsen 2 in das Behältnis eingebracht. Nach der Behandlung der einen Linsenfläche wird die Linse in der Beladestation gewendet und über die Schleuse erneut in das Behältnis 5 eingebracht. Die Halterung 1 für die Kontaktlinse kann aber auch um ihre Längsachse um mindestens 180° drehbar sein. Auf diese Weise kann die Kontaktlinse 2 gegenüber dem Laserstrahl gewendet werden, um die Front- und die Rückfläche der Linse mit dem Laserstrahl zu beaufschlagen. Das Behältnis 5 weist auch nicht dargestellte Entlüftungs- und Entkeimungsmittel auf. Derart ausgebildet kann das Innere des Behältnisses weitgehend keimfrei gehalten werden, sodass die Behandlung der Kontaktlinse in steriler Umgebung erfolgen kann. Selbstverständlich kann die gesamte Vorrichtung selbst in einem Sterilraum angeordnet sein. In diesem Fall erübrigt sich das Behältnis 5. Auch kann die Kontaktlinse 2 beispielsweise noch in einer Giessformhälfte enthalten sein, und die Halterung 1 zur Aufnahme der Giessformhälfte ausgebildet sein.

Zur Reinigung und Sterilisation der Front- und Rückfläche der Kontaktlinse 2 wird diese in der vorzugsweise automatisch drehbaren Halterung 1 im Strahlengang des Excimer-Lasers 3 angeordnet. Die Oberflächenreinigung und Sterilisation der Kontaktlinsen 2 erfolgt nun durch Beaufschlagen der Oberflächen der Kontaktlinsen 2 mit Laserstrahlung 10 einer bestimmten Wellenlänge, Energiedichte und Intensität. Dadurch werden die an der Front- bzw. Rückfläche der Kontaktlinse 2 anhaftenden Verunreinigungen ablativ entfernt und im selben Arbeitsgang gegebenfalls vorhandene Mikroorganismen abgetötet, inaktiviert oder gleichfalls photoablativ entfernt.

In bevorzugter Weise werden die Front- bzw. die Rückfläche der Kontaktlinse 2 gesamthaft mit dem Laserstrahl 10 beaufschlagt. Zu diesem Zweck wird der vom Excimer-Laser 3 kommende Laserstrahl mit Hilfe der Strahlaufweitmittel aufgeweitet. Durch geeignete Wahl der Wellenlänge des Laserlichtes, welche beim Excimer-Laser beispielsweise durch Wechsel oder Veränderung des Verstärkungsmediums gut einstellbar ist, seiner Energiedichte und Intensität können die Verunreinigungen und/oder Kontaminationen selektiv von der Linsenoberfläche abgetragen werden, ohne die Oberfläche der Kontaktlinse 2 zu beschädigen.

In einem alternativen Verfahren werden die Oberflächen der Kontaktlinse 2 vor der Oberflächenreinigung und Sterilisation von einem niederenergetischen Laserstrahl abgetastet, und die Verunreinigungen und Kontaminationen auf laserinterferometrische Weise detektiert und deren örtliche Verteilung auf der Kontaktlinse 2 registriert. Die Abtastung der Linsenoberflächen kann beispielsweise in einer separaten Oberflächeninspektionsstation mit Hilfe eines zusätzlichen Hilfslasers erfolgen, es kann aber auch der Excimer-Laser 3 dazu verwendet werden. Zum Zweck der Abtastung wird der Excimer-Laser 3 in einem niederenergetischen Modus betrieben. Die detektierten und abgespeicherten Koordinaten der Verunreinigungen werden an Fokussier- und Strahlablenkmittel weitergegeben, mit deren Hilfe der Arbeitslaser sodann auf die detektierten Verunreinigungen und Kontaminationen gelenkt wird, um diese abzutragen.

Anstatt den fokussierten Laserstrahl 10 über die Linsenoberfläche zu scannen, kann die Linsenoberfläche auch gesamthaft bestrahlt werden. Die nicht verunreinigten Bereiche der Linsenoberfläche werden dabei von einer bereichsweise bezüglich ihrer Transmission beeinflussbaren Maske, beispielsweise einer LCD-Maske, abgedeckt. Die Wellenlänge, die Energie und Intensität des Laserstrahls 10 sind dabei wieder derart bemessen, dass die Verunreinigungen und gegebenenfalls vorhandenen Kontaminationen selektiv abgetragen werden ohne die Oberfläche der Kontaktlinse 2 zu beschädigen.

Die auf den Kontaktlinsen oftmals anhaftenden Wachsrückstände sind im allgemeinen sehr dünn, üblicherweise weisen sie eine Dicke von weniger als 1 µm auf. In einer Verfahrensvariante werden bei ganzflächiger Bestrahlung der Kontaktlinsenfront- oder -rückfläche die Verfahrensparameter wie Wellenlänge des Laserlichtes, Energiedichte und Intensität des Laserstrahls derart gewählt, dass die Verunreinigungen und gegebenenfalls vorhandenen Kontaminationen zusammen mit einer dünnen Oberflächenschicht der Linse 2 abgetragen werden. Insbesondere werden die Verfahrensparameter dabei derart gewählt, dass in den nicht verunreinigten Bereichen nur eine Oberflächenschicht von maximal 1 µm Dicke abgetragen wird. Durch die vorübergehende Maskierwirkung der Verunreinigungen kommt es zwar zu einer geringfügigen Strukturierung der Linsenoberfläche. Die Stufenhöhen betragen aber maximal 1 µm und wirken sich nicht negativ auf die Funktionsweise der Kontaktlinse 2 aus. In Fig. 2 ist ein Schnitt einer Kontaktlinse 2 mit Verunreinigungen 6 dargestellt. Die strichlierte Linie 7 deutet den Frontflächenverlauf der Kontaktlinse 2 nach der Laserbehandlung an.

Diese Verfahrensvariante des ganzflächigen Materialabtrags von Kontaklinsenoberflächen bietet sich insbesondere auch bei Linsen 2 an, welche dünne Oberflächenschichten mit von Linsengrundmaterial verschiedenen Materialeingenschaften aufweisen. Derartige Linsen können beim sogenannten "full-mold"-Verfahren auftreten, bei dem die Kontaktlinse 2 in einem einstufigen Verfahren durch Polymerisation eines flüssigen Polymerisationsansatzes in einer Giessform hergestellt wird. Die dabei manchmal aufgrund von Oberflächeneffekten und Grenzflächeneffekten zwischen Linsenmaterial und Giessform bei der Polymerisation auftretenden Oberflächenschichten können ganzflächig entfernt werden. Hierbei wird das Linsenmaterial aus tieferen Schichten freigelegt, welches die gewünschten Bulk-Materialeigenschaften unverändert aufweist, ohne dabei die Grundgeometrie der Kontaktlinse 2 wesentlich zu beeinflussen.

Zur Durchführung des erfindungsgemässen Oberflächenreinigungs- und Sterilisationsverfahrens wird die Wellenlänge der Laserstrahlung zu 100 nm bis 350 nm eingestellt. Es versteht sich, dass Laserstrahlung der angegebenen Wellenlängenbereiche nicht nur mit Hilfe von Excimer-Lasern zu erreichen ist. Es kann auch ein Laser eingesetzt werden, der längere Wellenlängen liefert. Die gewünschten Wellenlängenbereiche erhält man bei einem derartigen Laser beispielsweise durch Frequenzverdopplung. Die Energiedichten werden von 0,1 J/cm² bis 10 J/cm² gewählt. Zur noch besseren Steuerung der Energiedeposition des Lasers wird dieser vorzugsweise im Pulsbetrieb betrieben.

Insgesamt erlaubt es das erfindungsgemässe Verfahren, die Oberflächen optischer Bauteile auf einfache, zeitsparende und kostengünstige Art zu reinigen und gegebenenfalls im selben oder in einem separaten Arbeitsgang zu sterilisieren. Insbesondere Kontaktlinsen aus harten hoch-sauerstoffdurchlässigen Materialien, die sehr temperaturempfindlich sind, lassen sich auf diese Weise einfach steriliseren. Die Bestrahlung kann in einer sterilen Umgebung erfolgen, sodass eine erneute Kontamination der Linsen ausgeschlossen werden kann. Die Verpackung der Linsen, insbesondere von Intraokularlinsen, kann unmittelbar anschliessend in derselben sterilen Umgebung erfolgen, so dass die Keimfreiheit der Linsen gewährleistet ist. Bei dem erfindungsgemässen Verfahren werden Reinigungsflüssigkeiten und Sterilisationsgase vermieden. Das Linsenmaterial wird schonend behandelt, Oberflächenbeschädigungen und Linsendeformation werden vermieden. Zudem ist das Verfahren auf einfache Weise automatisierbar und in bestehende Produktionsverfahren integrierbar.

## Patentansprüche

1. Verfahren zur gleichzeitigen Oberflächenreinigung und Sterilisation von optischen Bauteilen, insbesondere von Kontaktlinsen, bei welchem die Oberfläche des optischen Bauteils mit ultravioletter elektromagnetischer Strahlung beaufschlagt wird, dadurch gekennzeichnet, dass die Oberfläche des optischen Bauteils mit ultravioletter Laserstrahlung mit einer Wellenlänge von 100 nm bis 350 nm und einer Energiedichte von 0.1 J/cm² bis 10 J/cm² beaufschlagt wird, wodurch an den Oberflächen des Bauteils anhaftende Verunreinigungen durch die ultraviolette Laserstrahlung ablativ entfernt werden und gleichzeitig vorhandene Mikroorganismen abgetötet, inaktiviert und/oder photoablativ entfernt werden, ohne dabei die optischen Eigenschaften der bestrahlten Bauteile zu beeinträchtigen.

2. Verfahren nach Patentanspruch 1, dadurch gekennzeichnet, dass Verunreinigungen und/oder Kontaminationen an den Oberflächen des optischen Bauteils detektiert und selektiv abgetragen werden.

3. Verfahren nach Patentanspruch 1 oder 2, dadurch gekennzeichnet, dass die Oberflächen des optischen Bauteils, insbesondere die Front - und Rückfläche einer Kontaktlinse, ganzflächig mit ultravioletter Laserstrahlung beaufschlagt werden.

4. Verfahren nach einem der Patentansprüche 1-3, dadurch gekennzeichnet, dass die Wellenlänge und die Energiedichte der Laserstrahlung an die Art der abzutragenden Verunreingungen und/oder Kontaminationen derart angepasst werden, dass die optische Korrektur des optischen Bauteils im wesentlichen nicht verändert wird.

5. Verfahren nach einem der Patentansprüche 2-4, dadurch gekennzeichnet, dass die ultraviolette Laserstrahlung mit Hilfe von Masken auf die Verunreinigungen und/oder Kontaminationen konzentriert wird.

6. Verfahren nach einem der Patentansprüche 1-3, dadurch gekennzeichnet, dass die Verunreinigungen und/oder Kontaminationen gemeinsam mit einer Oberflächenschicht des optischen Bauteils abgetragen werden, deren Dicke durch Wahl der Wellenlänge und der Energiedichte der ultravioletten Laserstrahlung gesteuert wird.

7. Verfahren nach Patentanspruch 6, dadurch gekennzeichnet, dass eine Oberflächenschicht von maximal 1 µm Dicke abgetragen wird.

8. Verfahren nach einem der vorangehenden Patentansprüche, dadurch gekennzeichnet, dass die Bestrahlung des optischen Bauteils, insbesondere der Front- und Rückfläche einer Kontaktlinse, in steriler Umgebung erfolgt.

9. Verfahren nach Patentanspruch 8, dadurch gekennzeichnet, dass das optische Bauteil, insbesondere die Kontaktlinse, innerhalb eines transparenten Behältnisses angeordet wird, und dass das Bauteil nach Bestrahlung der einen Oberfläche, vorzugsweise der Frontfläche, automatisch gewendet wird, um die andere Oberfläche des Bauteils zu bestrahlen.

10. Verfahren einem der Patentansprüche 1 bis 9, dadurch gekennzeichnet, dass die ultraviolette Laserstrahlung gepulst wird.

11. Verfahren nach einem der Patentansprüche 1 bis 10, dadurch gekennzeichnet, dass als Quelle für die ultraviolette Laserstrahlung ein Excimer-Laser eingesetzt wird.

## Claims

1. A method of simultaneously surface-cleaning and sterilising optical components, especially contact lenses, in which the surface of the optical component is acted upon by ultraviolet electromagnetic radiation, which comprises causing ultraviolet laser radiation of a wavelength of from 100 nm to 350 nm and an energy density of from 0.1 J/cm² to 10 J/cm² to act on the surface of the optical component, so that impurities adhering to the surfaces of the component are removed ablatively by the ultraviolet laser radiation and at the same time any microorganisms present are killed, deactivated and/or removed photoablatively, without the optical properties of the irradiated components being impaired.

2. A method according to patent claim 1, which comprises detecting impurities and/or contaminations on the surfaces of the optical component and selectively removing them.

3. A method according to patent claim 1 or patent claim 2, which comprises causing the ultraviolet laser radiation to act on the entire face of the surfaces of the optical component, especially the front and rear faces of a contact lens.

4. A method according to any one of patent claims 1 to 3, which comprises adapting the wavelength and the energy density of the laser radiation to the type of impurity and/or contamination to be removed, in such a manner that the optical correction of the optical component is substantially unaltered.

5. A method according to any one of patent claims 2 to 4, which comprises concentrating the ultraviolet laser radiation on the impurities and/or contaminations by means of masks.

6. A method according to any one of patent claims 1 to 3, which comprises removing the impurities and/or contaminations together with a surface layer of the optical component, the thickness of which layer is controlled by the choice of wavelength and energy density of the ultraviolet laser radiation.

7. A method according to patent claim 6, which comprises removing a surface layer of no more than 1 µm thick.

8. A method according to any one of the preceding patent claims, which comprises carrying out the irradiation of the optical component, especially the front and rear faces of a contact lens, in a sterile environment.

9. A method according to patent claim 8, which comprises arranging the optical component, especially the contact lens, inside a transparent housing, and, after irradiating one surface, preferably the front face, turning the component round automatically in order to irradiate the other surface of the component.

10. A method according to any one of patent claims 1 to 9, which comprises pulsing the ultraviolet laser radiation.

11. A method according to any one of patent claims 1 to 10, which comprises using an excimer laser as the source of the ultraviolet laser radiation.

## Revendications

1. Procédé de nettoyage superficiel et de stérilisation simultanés de pièces optiques, en particulier de lentilles de contact, dans lequel la surface de la pièce optique est exposée à un rayonnement électromagnétique ultraviolet, caractérisé en ce que la surface de la pièce optique est exposée à un rayonnement laser ultraviolet ayant une longueur d'onde de 100 à 350 nm et une densité d'énergie de 0,1 à 10 J/cm², ce qui provoque l'enlèvement, par ablation par le rayonnement laser ultraviolet, des impuretés qui adhèrent aux surfaces de la pièce, et simultanément les micro-organismes présents sont tués, inactivés et/ou éliminés par photo-ablation, sans influer d'une manière négative sur les propriétés optiques des pièces exposées.

2. Procédé selon la revendication 1, caractérisé en ce que les saletés et/ou contaminations se trouvant sur les surfaces de la pièce optique sont détectées et sélectivement enlevées.

3. Procédé selon la revendication 1 ou 2, caractérisé en ce que les surfaces de la pièce optique, en particulier la surface frontale et la surface arrière d'une lentille de contact, sont exposées en pleine surface au rayonnement laser ultraviolet.

4. Procédé selon l'une des revendications 1 à 3, caractérisé en ce que la longueur d'onde et la densité d'énergie du rayonnement laser sont adaptées à la nature des saletés et/ou des contaminations à enlever, de telle sorte que la correction optique de la pièce optique ne subisse pas une modification trop importante.

5. Procédé selon l'une des revendications 2 à 4, caractérisé en ce que le rayonnement laser ultraviolet est concentré à l'aide de masques sur les impuretés et/ou contaminations.

6. Procédé selon l'une des revendications 1 à 3, caractérisé en ce que les saletés et/ou contaminations sont enlevées en même temps qu'une couche superficielle de la pièce optique, dont l'épaisseur est régulée par le choix de la longueur d'onde et de la densité d'énergie du rayonnement laser ultraviolet.

7. Procédé selon la revendication 6, caractérisé en ce qu'on enlève une couche superficielle ayant une épaisseur maximale de 1 µm.

8. Procédé selon l'une des revendications précédentes, caractérisé en ce que l'exposition de la pièce optique, en particulier de la surface frontale et de la surface arrière d'une lentille de contact, s'effectue dans un environnement stérile.

9. Procédé selon la revendication 8, caractérisé en ce que la pièce optique, en particulier la lentille de contact, est disposée à l'intérieur d'une enceinte transparente, et que la pièce, après exposition de l'une des surfaces, de préférence de la surface frontale, subit un retournement automatique, pour permettre l'exposition de l'autre surface de la pièce.

10. Procédé selon l'une des revendications 1 à 9, caractérisé en ce que le rayonnement laser ultraviolet est pulsé.

11. Procédé selon l'une des revendications 1 à 10, caractérisé en ce qu'on utilise en tant que source de rayonnement laser ultraviolet un laser Excimer.
